# EUROPÄISCHE PATENTSCHRIFT

(11) **EP 0 738 504 B1**
(45) Veröffentlichungstag und Bekanntmachung des Hinweises auf die Patenterteilung: **22.03.2000**
(21) Anmeldenummer: 95810263.4
(22) Anmeldetag: 20.04.1995
(51) Int. Cl.: A61F 2/38

(54) **Tibiaplattform zu einer Kniegelenkprothese**
Tibial tray for a knee joint prosthesis
Plateau tibial d'une prothèse de l'articulation du genou

(43) Veröffentlichungstag der Anmeldung: 23.10.1996
(73) Patentinhaber: Sulzer Orthopädie AG, 6340 Baar (CH)
(72) Erfinder: Schneider, Markus, CH-8580 Amriswil (CH); Moser, Walter, Dr., CH-3037 Herrenschwanden (CH)
(74) Vertreter: Trieblnig, Adolf

(56) Entgegenhaltungen:
- EP-A- 0 495 340
- US-A- 4 944 757
- US-A- 5 330 534

## Beschreibung

Die Erfindung handelt von einer Tibiaplattform zu einer Kniegelenkprothese mit einem Verankerungsteil zur Befestigung an einem Tibiaknochen und mit einer Gelenkauflage, welche über mindestens eine an ihr angeformte Federklinke mit dem Verankerungsteil durch Zurückfedern und Einschnappen verklinkbar ist, wobei in Rückfederrichtung der Federklinke eine Schulter an der Gelenkauflage angeformt ist.

Eine solche Ausführung ist in der Patentanmeldung EP-A-0 495 340 gezeigt, bei der eine Gelenkauflage über eine Federklinke am Herausfallen gehindert wird. Ein solches System schliess nicht aus, dass im verklinkten Zustand Kräfte in der Rückfederrichtung der Federklinke zwischen Verankerungsteil und Gelenkauflage auftreten, die eine wiederholte Rückfederung der Federklinke bewirken.

Aufgabe der Erfindung ist es, diesem Umstand abzuhelfen. Diese Aufgabe wird mit den Kennzeichen des unabhängigen Anspruches 1 gelöst, indem, zwischen Federklinke und Schulter ein Zwischenraum mit definiertem Abstand ausgebildet ist, und indem die Tibiaplattform einen Druckkörper aufweist, der bei verklinkter Gelenkauflage in den Zwischenraum einsetzbar ist und diesen ausfüllt, um ein Rückfedern der Federklinke zu verhindern.

Ein Vorteil dieser Anordnung ist, dass Druckkörper und die Federklinke im eingesetzten Zustand nur auf Druck und nicht auf Biegung beansprucht werden, wenn Kräfte in der Rückfederrichtung auftreten. Ein weiterer Vorteil ist, dass mit den einsetzbaren Druckkörpern die Lage der Federklinke überprüfbar wird, wenn sich der Druckkörper nur bei vollständig eingerasteter Federklinke einsetzen lässt. Dadurch dass am Druckkörper nur in einer Richtung, nämlich in der Rückfederrichtung der Federklinke Kräfte auftreten können, lässt sich dieser selbst in der Gelenkauflage durch eine Verklinkung sichern, die nicht durch wechselnde Druckkräfte beeinflusst ist. Da der Druckkörper im wesentlichen nur durch wechselnde Druckkräfte belastet wird, sind nicht nur Metalle sondern auch Kunststoffe als Werkstoff für den Druckkörper möglich.

Im folgenden ist ein Ausführungsbeispiel für die Erfindung aufgeführt. Es zeigen:
- Fig. 1: schematisch eine Verklinkung einer Gelenkauflage mit einem Verankerungsteil;
- Fig. 2: schematisch die Draufsicht auf eine Gelenkauflage, die mit einem Druckkörper verriegelbar ist;
- Fig. 3: schematisch einen Querschnitt längs des Zwischenraumes in Figur 2 für den Druckkörper;
- Fig. 4: schematisch einen vergrösserten Längsschnitt durch die Gelenkauflage in Fig. 2 im Bereich der Federklinke, und
- Fig. 5: schematisch die vergrösserte Ansicht eines als Agraffe ausgeführten Druckkörpers.

In den Figuren ist eine C-förmige Agraffe als Druckkörper 8 gezeigt, die in einer Tasche 15 eine Gelenkauflage 4 einbringbar ist und die selbst über eine Verklinkung in dieser Tasche 15 gegen Herausfallen sicherbar ist. Dabei wirken die beiden Schenkel der Agraffe quer zu ihrer Federrichtung als Distanzhalter zwischen einer Federklinke 5 und einer Schulter, welche in der Gelenkauflage 4 angeformt sind. Es entsteht so eine einfache und in der Gelenkauflage 4 versenkte Sicherung, die ein Rückfedern der eingerasteten Federklinke 5 verhindert und nur auf Druck beansprucht ist. Im montierten Zustand wird die Federklinke 5 selbst keinen wechselnden Biegebelastungen ausgesetzt.

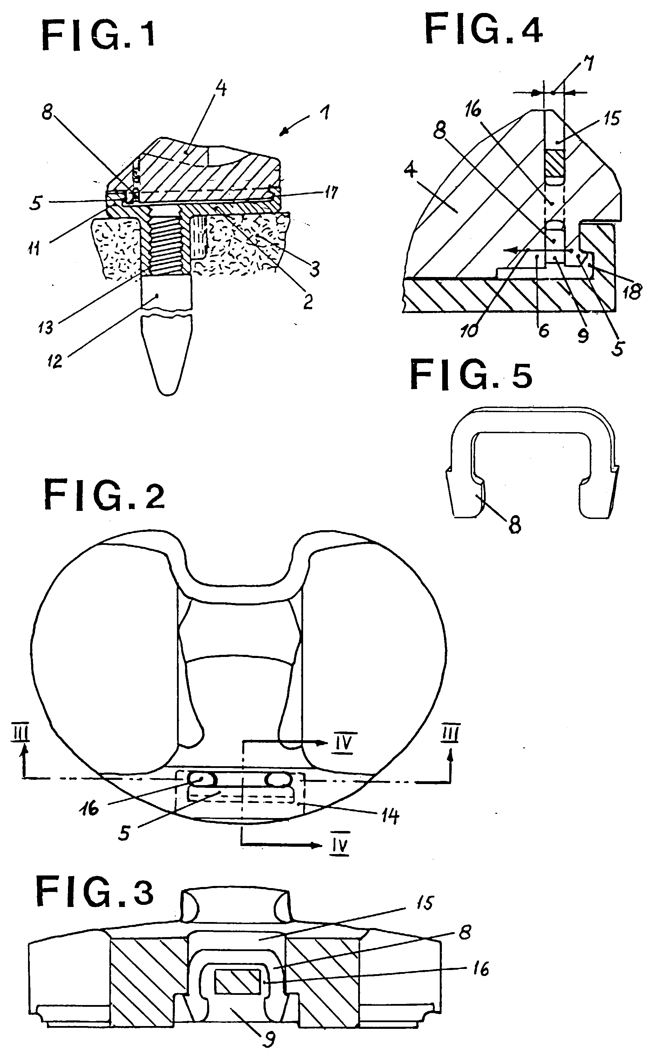

Eine Tibiaplattform 1 (Fig. 1) besteht aus einem Verankerungsteil 2 aus Metall, in den ein Verankerungszapfen 12 über ein Gewinde 13 eingeschraubt ist, um diesen im Tibiaknochen 3 für ein künstliches Kniegelenk zu verankern. Der Verankerungsteil besitzt einen nach oben vorstehenden Rand 11, der auf der Innenseite Bereiche mit Nuten aufweist. Posterior bilden diese Nuten mit der Gelenkauflage 4 ein offenes Scharnier 17 während anterior (Fig. 4) eine Federklinke 5, die aus der Gelenkauflage 4 herausgearbeitet ist, in der Nute 18 einrastet, um die Gelenkauflage im Verankerungsteil zu halten. Beim Einrasten wird die Federklinke 5 in einer Rückfederrichtung 10 zurückgestossen und könnte bei ungünstigen Angriffskräften auch im eingerasteten Zustand wechselnden Biegebelastungen ausgesetzt sein. Aus diesem Grund ist in Rückfederrichtung 10 hinter der Federklinke 5 eine Tasche 15 herausgearbeitet, die über zwei Verbindungsöffnungen 16 in einen Zwischenraum 9 mündet, der durch einen genauen Abstand 7 zwischen der Federklinke 5 und einer in Rückfederrichtung gegenüberliegenden Schulter 6 definiert ist. Ein Druckkörper 8 in Form einer Agraffe entspricht mit seiner Dicke dem Abstand 7 und lässt sich von oben in die Tasche 15 einsetzen bis er einerseits quer zur Rückfederrichtung 10 verklinkt ist und andererseits den Zwischenraum 9 ausfüllt, um ein Rückfedern und damit eine Biegebelastung von der Federklinke 5 zu verhinden. Die Agraffe ist ausserhalb der eigentlichen Gelenkführungsfläche von oben einsetzbar und versenkbar. Sie lässt sich, da sie praktisch nur auf Druck beansprucht ist in verschiedenen Werkstoffen ausführen. Bei metallischer Ausführung kann ihre Lage auf einem Röntgenbildschirm kontrolliert werden.

## Patentansprüche

1. Tibiaplattform (1) zu einer Kniegelenkprothese mit einem Verankerungsteil (2) zur Befestigung an einem Tibiaknochen (3) und mit einer Gelenkauflage (4), welche über mindestens eine an ihr angeformte Federklinke (5) mit dem Verankerungsteil (2) durch Zurückfedern und Einschnappen verklinkbar ist, wobei in Rückfederrichtung (10) der Federklinke (5) eine Schulter (6) an der Gelenkauflage (4) angeformt ist, dadurch gekennzeichnet, dass zwischen Federklinke (5) und Schulter (6) ein Zwischenraum (9) mit definiertem Abstand (7) ausgebildet ist, und dass die Tibiaplattform einen Druckkörper (8) aufweist, der bei verklinkter Gelenkauflage (4) in den Zwischenraum (9) einsetzbar ist und diesen ausfüllt, um ein Rückfedern der Federklinke (5) zu verhindern.

2. Tibiaplattform (1) nach Anspruch 1, dadurch gekennzeichnet, dass der Druckkörper (8) seinerseits an der Gelenkauflage (4) gegen Herausfallen sicherbar ist.

3. Tibiaplattform (1) nach Anspruch 2, dadurch gekennzeichnet, dass der Druckkörper (8) als C-förmige Agraffe in der Gelenkauflage (4) verklinkbar ist.

## Claims

1. Tibia platform (1) for a knee joint prosthesis comprising an anchoring part (2) for the attachment to a tibia bone (3) and a joint support (4) which can be latched via at least one spring pawl (5) formed on it to the anchoring part (2) by resilient deflection and snapping into place, wherein a shoulder (6) is formed on the joint support (4) in the spring-back direction (10) of the spring catch (5). characterised in that an intermediate space (9) with a defined spacing (7) is formed between the spring catch and the shoulder (6); and in that the tibia platform has a compression member (8) which can be inserted into the intermediate space (9) when the joint support (4) is latched and fills it out in order to prevent springing back of the spring catch (5).

2. Tibia platform (1) in accordance with claim 1, characterised in that the compression member (8) can in turn be secured on the joint support (4) against falling out of place.

3. Tibia platform (1) in accordance with claim 2, characterised in that the compression member (8) can be latched in the form of a C-shaped clip in the joint support (4).

## Revendications

1. Plateforme tibiale (1) pour une prothèse d'articulation du genou avec une pièce d'ancrage (2) destinée à être fixée à un os tibial (3) et avec un appui d'articulation (4) qui peut être enclenché par au moins un cliquet à ressort (5) rapporté par formage sur lui avec la pièce d'encrage (2) par un rebondissement et enclenchement, où il est rapporté par formage dans la direction de rebondissement (10) du cliquet à ressort (5) un épaulement (6) sur l'appui d'articulation (4), caractérisée en ce qu'il est réalisé entre le cliquet à ressort (5) et l'épaulement (6) un espace intermédiaire (9) d'un écart défini (7), et que le plateau tibial présente un corps de pression (8) qui, lorsque l'appui d'articulation (4) est enclenché, peut être placé dans l'espace intermédiaire (9) et remplit celui-ci pour empêcher un rebondissement du cliquet à ressort (5).

2. Plateforme tibiale (1) selon la revendication 1, caractérisé en ce que le corps de pression (8) peut être assuré de son côté à l'appui d'articulation (4) à l'encontre d'une chute.

3. Plateau tibial (1) selon la revendication 2, caractérisé en ce que le corps de pression (8) en tant qu'agrafe en forme de C, peut être enclenché dans l'appui d'articulation (4).
